# EUROPÄISCHE PATENTSCHRIFT

(11) **EP 0 589 903 B1**
(45) Veröffentlichungstag und Bekanntmachung des Hinweises auf die Patenterteilung: **09.09.1998**
(21) Anmeldenummer: 92909823.4
(22) Anmeldetag: 08.05.1992
(51) Int. Cl.: C07D 277/40, C07D 417/12, A61K 31/425, A61K 31/435

(54) **AMINOALKYLSUBSTITUIERTE 5-MERCAPTOTHIAZOLE, IHRE HERSTELLUNG UND VERWENDUNG**
AMINOALKYL-SUBSTITUTED 5-MERCAPTOTHIAZOLES, THEIR PREPARATION AND THEIR USE
5-MERCAPTOTHIAZOLES A SUBSTITUANT AMINOALKYLE, LEUR PREPARATION ET LEUR UTILISATION

(30) Priorität: 15.06.1991 DE 4119756
(43) Veröffentlichungstag der Anmeldung: 06.04.1994
(73) Patentinhaber: BASF Aktiengesellschaft, 67063 Ludwigshafen (DE)
(72) Erfinder: RENDENBACH-MUELLER, Beatrice, D-6701 Waldsee (DE); UNGER, Liliane, D-6700 Ludwigshafen (DE); TESCHENDORF, Hans-Juergen, D-6724 Dudenhofen (DE)
(86) Internationale Anmeldenummer: EP9201004
(87) Internationale Veröffentlichungsnummer: WO9222540

(56) Entgegenhaltungen:
- EP-A- 0 345 533
- EP-A- 0 409 048
- EP-A- 0 412 404

## Beschreibung

Die Erfindung betrifft neue aminoalkylsubstituierte 5-Mercaptothiazole, deren Herstellung sowie deren Verwendung zur Bekämpfung von Krankheiten.

In der US-PS 3 717 651 werden u.a. 5-mercapto-substituierte Thiazole beschrieben, die herbizide Eigenschaften besitzen.

Es wurde nun gefunden, daß sich aminoalkylsubstituierte 5-Mercaptothiazole der Formel I 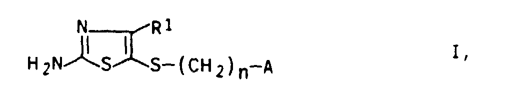 worin
- R¹: H, C₁-C₅-Alkyl, Phenyl, das gegebenenfalls durch Halogen, C₁-C₅-Alkyl oder C₁-C₅-Alkoxy substituiert ist, sowie Thienyl,
- n: eine ganze Zahl von 2 bis 6,
- A: NR²R³, worin R² und R³, die gleich oder verschieden sein können, Wasserstoff, C₁-C₅-Alkyl, das gegebenenfalls durch Phenyl oder Thienyl substituiert sein kann, bedeuten, oder eine der Gruppen 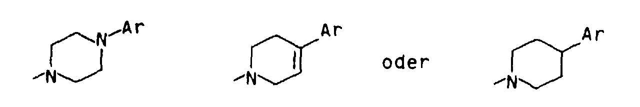 wobei Ar für einen gegebenenfalls einfach durch C₁-C₅-Alkyl, C₁-C₅-Alkoxy, Halogen, Nitro, Hydroxy, Trifluormethyl oder Cyano substituierten Phenylring, einen Pyridyl-, Pyrimidinyl- oder Thienylrest steht,
bedeuten, sowie deren Salze mit physiologisch verträglichen Säuren gut zur Bekämpfung von Krankheiten eignen.

zugsweise in der Formel I bedeutet R¹ vorzugsweise H, C₁-C₅-Alkyl, A vor- 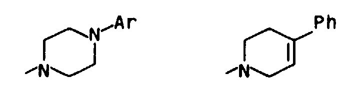 oder durch Phenyl oder Thienyl substituiertes C₁-C₅-Alkyl und n vorzugsweise die Zahl 2 oder 3.

Die Verbindungen der Formel I lassen sich herstellen, indem man
a) ein 5-Mercaptothiazol der Formel II 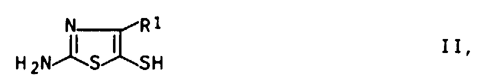 in der R¹ wie eingangs definiert ist, oder ein Salz dieser Verbindung mit und einem ω-X-Alkylamin der Formel III

   X-(CH₂)ₙ-A III,

   in der A und n wie eingangs definiert sind, und X für eine Abgangsgruppe wie Chlor, Brom oder R⁴SO₂O- steht [R⁴ = C₁-C₄-Alkyl oder gegebenenfalls durch C₁-C₃-Alkyl oder Halogen substituiertes Phenyl] umsetzt oder
b) ein ω-alkylsubstituiertes 5-Mercaptothiazol der Formel IV 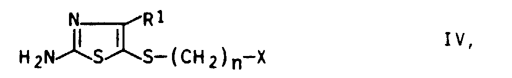 in der R¹, n und X die angegebenen Bedeutungen haben, oder ein Salz dieser Verbindungen mit einem Amin der Formel V

   HA V ,

   in der A wie eingangs definiert ist, umsetzt oder
c) ein ω-Mercaptoalkylamin der Formel VI

   HS-(CH₂)ₙ-A VI,

   in der A und n wie eingangs definiert sind, mit einem 5-Y-substituierten Thiazol der Formel VII 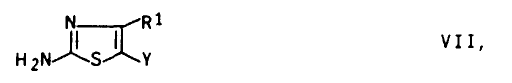 in der R¹ wie eingangs definiert ist, und Y Chlor oder Brom bedeutet, oder mit einem halogenwasserstoffsauren Salz dieser Verbindungen umsetzt
und die so erhaltenen Verbindungen gegebenenfalls in ihre Salze mit physiologisch verträglichen Säuren überführt.

Im Falle des Verfahrens a) erfolgen die Umsetzungen vorzugsweise in einem Lösungsmittel bei Temperaturen zwischen Raumtemperatur und der Siedetemperatur des verwendeten Lösungsmittels, gegebenenfalls in Gegenwart eines säurebindenden Mittels. Als Lösungsmittel können beispielsweise Dimethylformamid, oder Ketone wie Aceton oder Butanon verwendet werden, als säurebindende Mittel anorganische Basen wie Natrium- oder Kaliumcarbonat oder tertiäre organische Basen wie Triethylamin oder Pyridin. Letztere können, im überschuß verwendet, gleichzeitig als Lösungsmittel dienen.

Die Isolierung des Rohprodukts erfolgt in an sich üblicher Weise, beispielsweise durch Filtration, Abdestillieren des Lösungsmittels oder Extraktion aus dem Reaktionsgemisch. Die Reinigung der erhaltenen Verbindung erfolgt in üblicher Weise, beispielsweise durch Umkristallisieren aus einem Lösungsmittel, Chromatographie oder überführen in eine Säureadditionsverbindung.

Die als Ausgangsstoffe verwendeten 5-Mercaptothiazole der Formel II sind literaturbekannt oder können durch Behandlung eines Thiazols der Formel VIII, 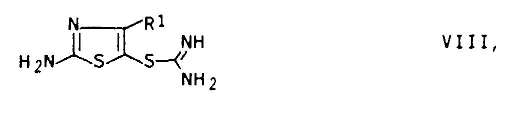 in der R¹ wie eingangs definiert ist, oder eines halogenwasserstoffsauren Salzes dieser Verbindungen mit Basen, wie wäßriger Ammoniaklösung oder Natronlauge, gewünschtenfalls auch in einem Zweiphasengemisch, hergestellt werden. Eine andere Synthesemöglichkeit stellt die Umsetzung der Thiazole der Formel VII mit anorganischen Sulfiden, wie Kaliumhydrogensulfid, dar. Die Mercaptothiazole der Formel II müssen zur weiteren Umsetzung nicht unbedingt isoliert werden. So ist es beispielsweise auch möglich, die erfindungsgemäßen Verbindungen der Formel I durch Behandlung eines Gemisches der Thiazole der Formel VIII und der ω-X-Alkylamine der Formel III mit einer Base zu erhalten, wobei das in situ gebildete Mercaptothiazol der Formel II direkt mit den Alkylaminen der Formel III reagiert.

Im Falle des Verfahrens b) erfolgen die Umsetzungen in der Schmelze, gewünschtenfalls auch in Gegenwart eines Lösungsmittels, wie Ethylacetat, Tetrahydrofuran, Dimethylformamid, Dimethoxyethan, Toluol oder Xylol, bei Temperaturen zwischen Raumtemperatur und dem Siedepunkt des verwendeten Lösungsmittels, vorzugsweise in Gegenwart einer Base, wie Natriummethylat, Natriumethylat, Natriumhydrid, Natrium carbonat, Kaliumcarbonat, oder eines Amins, wie Pyridin. Gegebenenfalls kann auch die Aminkomponente IV im überschuß als Reagenz, Base und Lösungsmittel fungieren.

Die Umsetzungen nach Verfahren c) erfolgen in einem Lösungsmittel bei Temperaturen zwischen Raumtemperatur und der Siedetemperatur des verwendeten Lösungsmittels. Geeignete Lösungsmittel sind beispielsweise Dimethylformamid, Dimethoxyethan, Tetrahydrofuran oder ein Keton, wie Aceton oder Butanon. Günstig ist der Zusatz einer Base, wie z.B. Natrium- oder Kaliumcarbonat, Natriumhydroxid, oder eines tertiären Amins, wie Pyridin oder Triethylamin.

Die als Ausgangsstoffe verwendeten Thiazole der Formel VII und Mercaptoalkylamine der Formel VI sind teilweise literaturbekannt oder können nach bekannten Methoden hergestellt werden.

Gegebenenfalls werden die erhaltenen erfindungsgemäßen Verbindungen in ihre Säureadditionsalze mit physiologisch verträglichen Säuren überführt. Als übliche physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere können aus Fortschritte der Arzneimittelforschung Bd. 10, S. 224 f., Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, einem halogenierten Kohlenwasserstoff, wie Methylenchlorid, einem Ether, wie Methyl-t-butylether oder Diisopropylether, einem Keton, wie Aceton oder Butanon, oder einem Ester, wie Essigsäureethylester, erhalten. Zur besseren Kristallabscheidung können auch Mischungen der genannten Lösungsmittel verwendet werden. Darüber hinaus können pharmazeutisch vertretbare wäprige Lösungen von Säureadditonsverbindungen der erfindungsgemäßen Verbindungen I durch Auflösen der freien Basen in einer wäßrigen Säurelösung hergestellt werden.

Die erfindungsgemäßen Verbindungen eignen sich zur Bekämpfung von Krankheiten, insbesondere zur Behandlung von Erkrankungen des zentralen Nervensystems (z.B. Parkinsonismus, Schizophrenie) sowie von erhöhtem Blutdruck. Sie weisen insbesondere wertvolle Eigenschaften als Dopaminagonisten, teilweise mit Selektivität für präsynaptische Dopaminrezeptoren, oder als Dopaminantagonisten auf. Die Verbindungen der Formel I zeigen Affinität zum Dopamin-Rezeptor in Rezeptorbindungstests; sie inhibieren die Motilität an Mäusen (gemessen in Lichtschrankenkäfigen) und beeinflussen das Rotationsverhalten an Ratten mit einseitigen 6-Hydroxydopamin-Läsionen der Substantia nigra (Brain Research 24, 485-493, 1970).

Die Wirkungen der neuen Verbindungen lassen sich in Rezeptorbindungstests zeigen:

Striata von Ratten (Sprague Dawley, Charles River) wurden sofort nach Entnahme in 0,32 M Saccharoselösung (0°C) homogenisiert. Das Homogenat wurde filtriert über Gaze, das Filtrat bei 1000 g zentrifugiert (5 min bei 4°C) und der resultierende überstand bei 40000 g zentrifugiert (10 min, 4°C). Der Rückstand (Membranen) wurde in Inkubationspuffer (50 mM Tris-HCl, 1 mM MgCl₂ und 0,1 % Ascorbinsäure, pH 7,4) aufgenommen und 20 min bei 37°C inkubiert. Anschließend wurde durch Resuspension und Rezentrifugation 2x mit Inkubationspuffer gewaschen. Die Membranen wurden portionsweise in flüssigem Stickstoff eingefroren.

Die Testansätze (1 ml) setzten sich zusammen aus Membranen (380 µg Protein), 1 nM ³H-ADTN (NEN, Dreieich Germany, spez. Radioaktivität 1,4 TBq/mmol) und 0,1 µM SCH 23390 (totale Bindung) oder a) mit zusätzlich 50 nM Spiperon (unspezifische Bindung) oder b) mit Prüfsubstanz. Die Ansätze wurden als Triplikate hergestellt.

Nach beendeter Inkubation (40 min bei 25°C) wurden die Ansätze über Glasfaserfilter (Whatman GF/B) filtriert und kurz mit eiskaltem Waschpuffer (Tris-HCl, pH 7,4) gewaschen. Die auf den Filtern zurückgehaltene Radioaktivität wurde mittels Flüssigkeitszintillationsmessung bestimmt. Die unspezifische Bindung betrug ca. 40 - 50 % der totalen Bindung.

Die Auswertung der Kompetitionskurven und die Bestimmung der Dissoziationskonstante erfolgte über iterative nichtlineare Regressionsanalyse in Anlehnung an das Programm "Ligand" (Muson und Rodbard: Anal. Biochem. 107, 220, 1980).

Affinität der Prüfsubstanzen zum Dopamin-D₂-Rezeptor

| Beispiel | Ki (nM) |
|---|---|
| 1 | 12 |
| 2 | 3 |
| 3 | 19 |
| 5 | 18 |
| 7 | 9 |

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 500 mg pro Patient und Tag bei oraler Gabe und etwa 1 bis 100 mg pro Patient und Tag bei parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließregulierlmitteln, Weichmachern, Netzmitteln, Dispergiermitteln, Emulgatoren, Lösungsmitteln, Retardierungsmitteln, Antioxidatien und/oder Treibgasen verarbeitet werden (vgl. H. Sucker et al: Pharmazeutische Technologie, Thieme-Verlag, Stuttgart, 1978). Die so erhaltenen Applikationen enthalten den Wirkstoff normalerweise in einer Menge von 1 bis 99 Gew.-%.

Die folgenden Beispiele sollen die Erfindung näher erläutern:

### Beispiele

1. 2-Amino-4-methyl-5-[2-(4-phenylpiperidinyl)-ethylthio]-thia-zol-hydrochlorid
   9,65 g 2-Amino-5-brom-4-methylthiazol, 11,1 g 2-(4-Phenyl-piperidin-1-yl)-ethylmercaptan und 20,7 g Kaliumcarbonat wurden in 50 ml Dimethylformamid 30 Minuten bei 80°C gerührt. Die Reaktionsmischung wurde auf Eiswasser gegossen, mit Methylenchlorid extrahiert und die organische Phase mit Wasser gewaschen, getrocknet und eingeengt. Der Rückstand wurde chromatographisch (SiO₂; CH₂Cl₂, CH₃OH (0 - 20 %)) gereinigt. Durch Lösen der freien Base in Methanol und Zugabe von etherischer HCI wurde das Hydrochlorid hergestellt. Ausbeute 4,8 g (26%); Fp.: 205 - 210°C.
2. Analog wurden hergestellt:
   2-Amino-5- [2-(1, 2,3,6-tetrahydro-4-phenylpyridinyl)-ethyl-thio]-4-methyl-thiazol-hydrochlorid
   Ausbeute: 17 %; Fp.: 204 - 206°C
3. 2-Amino-4-methyl-5-[2-(4-phenylpiperazinyl)-ethylthio]-thiazol-hydrochlorid
   Ausbeute: 20 %; Fp.: 235 - 237°C
4. 2-Amino-5-[2-(4-phenylpiperazinyl)ethylthio]-thiazol-hydro-chlorid
   Ausbeute: 15 %; Fp.: ab 55°C (Zersetzung)
5. 2-Amino-4-methyl-5-[2-(N-phenethyl-N-propylamino)-ethyl-thio]-thiazolhydrochlorid
   Ausbeute: 25 % Fp.: 104°C
6. 2-Amino-4-methyl-5-[2-(4-pyridin-2-ylpiperazinyl)-ethyl-thio]-thiazoltartrat
   Ausbeute: 20 % Fp.: 122 - 123°C
7. 2-Amino-4-methyl-5-[3-(N-phenethyl-N-propylamino)-propyl-thio]-thiazol-tartrat
   Ausbeute: 31 %; Fp.: 118 - 120°C.

Beispiele für galenische Applikationsformen:
A) Auf einer Tablettenpresse werden in üblicher Weise Tabletten folgender Zusammensetzung gepreßt.
   40 mg Substanz des Beispiels 1
   120 mg Maisstärke
   13,5 mg Gelatine
   45 mg Milchzucker
   2,25 mg Aerosil^{®} (chemisch reine Kieselsäure in submikroskopisch feiner Verteilung)
   6,75 mg Kartoffelstärke (als 6 %iger Kleister)
B)
   20 mg Substanz des Beispiels 4
   60 mg Kernmasse
   60 mg Verzuckerungsmasse
   Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Kalk. Die so hergestellten Dragees werden anschließend mit einem magensaftresistenten überzug versehen.
C) 10 g Substanz des Beispiels 2 werden in 5000 ml Wasser unter Zusatz von NaCl gelöst und mit 0,1N NaOH auf pH 6,0 eingestellt, so daß eine blutisotonische Lösung entsteht. Jeweils 1 ml dieser Lösung wird in Ampullen gefüllt und sterilisiert.

## Patentansprüche

1. Aminoalkylsubstituierte 5-Mercaptothiazole der Formel I 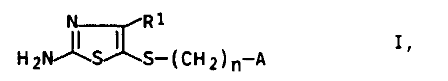 worin
R¹ H, C₁-C₅-Alkyl, Phenyl, das gegebenenfalls durch Halogen, C₁-C₅-Alkyl oder C₁-C₅-Alkoxy substituiert ist, sowie Thienyl,
n eine ganze Zahl von 2 bis 6,
A NR²R³, worin R² und R³, die gleich oder verschieden sein können, Wasserstoff, C₁-C₅-Alkyl, das gegebenenfalls durch Phenyl oder Thienyl substituiert sein kann, bedeuten, oder
eine der Gruppen 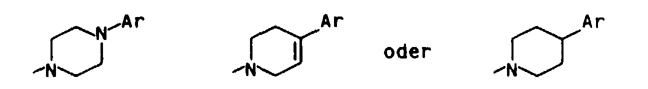 wobei Ar für einen gegebenenfalls einfach durch C₁-C₅-Alkyl, C₁-C₅-Alkoxy, Amino, Halogen, Nitro, Hydroxy, Trifluormethyl oder Cyano substituierten Phenylring, einen Pyridyl-, Pyrimidinyl- oder Thienylrest steht,
bedeuten, sowie deren Salze mit physiologisch verträglichen Säuren.

2. Verfahren zur Herstellung aminoalkylsubstituierter 5-Mercaptothiazole der Formel I gemäß Anspruch 1, dadurch gekennzeichnet, daß man
a) ein 5-Mercaptothiazol der Formel II 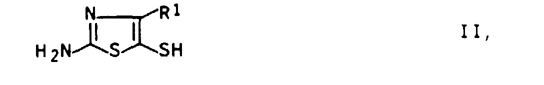 in der R¹ wie eingangs definiert ist, oder ein Salz dieser Verbindung mit einem ω-X-Alkylamin der Formel III
X-(CH₂)ₙ-A III,
in der A und n wie eingangs definiert sind und X für eine Abgangsgruppe wie Chlor, Brom oder R⁴SO₂O- steht [R⁴ = C₁-C₄-Alkyl oder gegebenenfalls durch C₁-C₃-Alkyl oder Halogen substituiertes Phenyl], oder einem Salz dieser Verbindung umsetzt oder
b) ein ω-X-alkylsubstituiertes 5-Mercaptothiazol der Formel IV 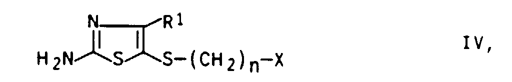 in der R¹, n und X die angegebenen Bedeutungen haben, oder ein Salz dieser Verbindungen mit einem Amin der Formel V
HA V,
in der A wie eingangs definiert ist, umsetzt oder
c) ein ω-Mercaptoalkylamin der Formel VI
HS-(CH₂)ₙ-A VI,
in der A und n wie eingangs definiert sind, mit einem 5-Y-substituierten Thiazol der Formel VII 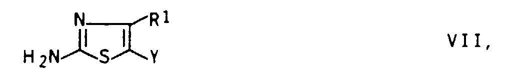 in der R¹ wie eingangs definiert ist, und Y Chlor oder Brom bedeutet, oder mit einem halogenwasserstoffsauren Salz dieser Verbindungen umsetzt
und die so erhaltenen Verbindungen gegebenenfalls in ihre Salze mit physiologisch verträglichen Säuren überführt.

3. Aminoalkylsubstituierte 5-Mercaptothiazole der Formel I gemäß Anspruch 1 zur Verwendung bei der Bekämpfung von Krankheiten.

4. Verwendung eines 5-Mercaptothiazols der Formel I gemäß Anspruch 1 zur Herstellung eines Arzneimittels gegen Erkrankungen des zentralen Nervensystems oder Erkrankungen, die mit erhöhtem Blutdruck einhergehen.

## Claims

1. An aminoalkyl-substituted 5-mercaptothiazole of the formula I 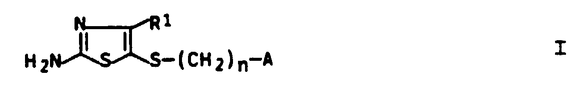 where
R¹ is H, C₁-C₅-alkyl, phenyl which is unsubstituted or substituted by halogen, C₁-C₅-alkyl or C₁-C₅-alkoxy, or is thienyl,
n is an integer from 2 to 6,
A is NR²R³ where R² and R³, which can be identical or different, are each hydrogen, C₁-C₅-alkyl, which is unsubstituted or substituted by phenyl or thienyl, or is 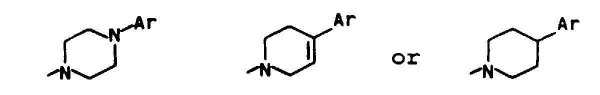 where Ar is phenyl which is unsubstituted or monosubstituted by C₁-C₅-alkyl, C₁-C₅-alkoxy, amino, halogen, nitro, hydroxyl, trifluoromethyl or cyano, or is pyridyl, pyrimidinyl or thienyl,
and its salts with physiologically tolerated acids.

2. A process for preparing aminoalkyl-substituted 5-mercaptothiazoles of the formula I as claimed in claim 1, which comprises
a) reacting a 5-mercaptothiazole of the formula II 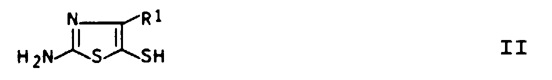 where R¹ is as defined above, or a salt of this compound, with an ω-X-alkylamine of the formula III
X-(CH₂)ₙ-A III
where A and n are as defined above, and X is a leaving group such as chlorine, bromine or R⁴SO₂O-[R⁴ = C₁-C₄-alkyl or phenyl which is unsubstituted or substituted by C₁-C₃-alkyl or halogen], or a salt of this compound, or
b) reacting an ω-alkyl-substituted 5-mercaptothiazole of the formula IV 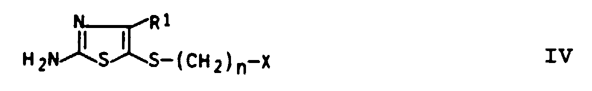 where R¹, n and X have the stated meanings, or a salt of this compound, with an amine of the formula V
HA V
where A is as defined above, or
c) reacting an ω-mercaptoalkylamine of the formula VI
HS- (CH₂)ₙ-A VI
where A and n are as defined above, with a 5-Y-substituted thiazole of the formula VII 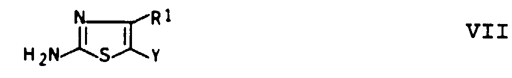 where R¹ is as defined above, and Y is chlorine or bromine, or with a hydrohalic acid salt of this compound,
and converting the resulting compounds where appropriate into their salts with physiologically tolerated acids.

3. An aminoalkyl-substituted 5-mercaptothiazole of the formula I as claimed in claim 1 for use for controlling diseases.

4. The use of a 5-mercaptothiazole of the formula I as claimed in claim 1 for producing a drug for disorders of the central nervous system or disorders associated with elevated blood pressure.

## Revendications

1. 5-mercaptothiazoles à substituants aminoalkyle, répondant à la formule I 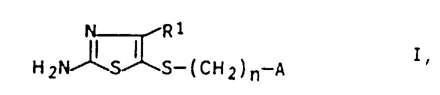 dans laquelle
R¹ représente H, un groupe alkyle en C1-C5, un groupe phényle éventuellement substitué par des halogènes, des groupes alkyle en C1-C5 ou alcoxy en C1-C5, ou un groupe thiényle,
n est un nombre entier allant de 2 à 6,
A représente NR²R³, R² et R³, ayant des significations identiques ou différentes, représentant l'hydrogène, un groupe alkyle en C1-C5 éventuellement substitué par un groupe phényle ou thiényle, ou bien
l'un des groupes 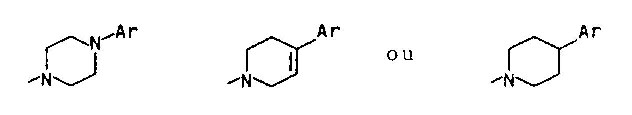 dans lesquels Ar représente un cycle phényle portant éventuellement un substituant alkyle en C1-C5, alcoxy en C1-C5, amino, halogéno, nitro, hydroxy, trifluorométhyle ou cyano, un radical pyridyle, pyrimidinyle ou thiényle,
et leurs sels d'acides acceptables pour l'usage pharmaceutique.

2. Procédé de préparation des 5-mercaptothiazoles à substituants aminoalkyle de formule I selon la revendication 1, caractérisé par le fait que
a) on fait réagir un 5-mercaptothiazole de formule II 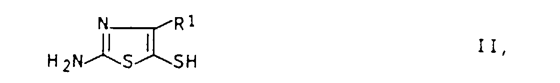 dans laquelle R¹ a les significations indiquées ci-dessus, ou un sel de ce composé, avec une oméga-X-alkylamine de formule III
X-(CH₂)ₙ-A III
dans laquelle A et n ont les significations indiquées ci-dessus et X représente un substituant éliminable tel que le chlore, le brome ou R⁴SO₂O (R⁴ = alkyle en C1-C4 ou phényle éventuellement substitué par alkyle en C1-C3 ou halogène), ou un sel de ce composé, ou bien
b) on fait réagir un 5-mercaptothiazole à substituant oméga-X-alkyle, de formule IV 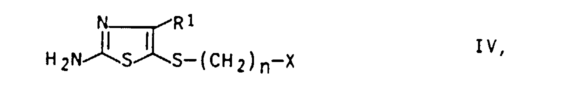 dans laquelle R¹, n et X ont les significations indiquées ci-dessus, ou un sel de l'un de ces composés, avec une amine de formule V
HA V
dans laquelle A a les significations indiquées ci-dessus, ou bien
c) on fait réagir une oméga-mercaptoalkylamine de formule VI
HS-(CH₂)ₙ-A VI
dans laquelle A et n ont les significations indiquées ci-dessus, avec un thiazole à substituant 5-Y, répondant à la formule VII 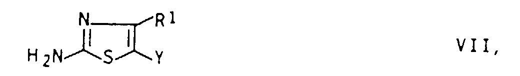 dans laquelle R¹ a les significations indiquées ci-dessus et Y représente le chlore ou le brome, ou avec un halogénhydrate de l'un de ces composés,
et, le cas échéant, on convertit les composés ainsi obtenus en leurs sels d'acides acceptables pour l'usage pharmaceutique.

3. 5-mercaptothiazoles à substituants aminoalkyle de formule I selon la revendication 1 pour l'utilisation dans le traitement de maladies.

4. Utilisation d'un 5-mercaptothiazole de formule I selon la revendication 1 pour la préparation de médicaments prévus pour le traitement des maladies du système nerveux central ou des maladies associées à une hypertension.
